Europäisches Patentamt

European Patent Office

Office européen des brevets

(19)

(11) Veröffentlichungsnummer: **0 263 292 B1**

# EUROPÄISCHE PATENTSCHRIFT

(12)

(45) Veröffentlichungstag der Patentschrift:
05.06.91 Patentblatt 91/23

(51) Int. Cl.⁵: **A61F 2/46**

(21) Anmeldenummer: 87112396.4

(22) Anmeldetag: 26.08.87

(54) Instrument zum Fräsen einer Krümmung in einem Femurknochen.

(30) Priorität: 02.10.86 CH 3937/86

(43) Veröffentlichungstag der Anmeldung:
13.04.88 Patentblatt 88/15

(45) Bekanntmachung des Hinweises auf die
Patenterteilung:
05.06.91 Patentblatt 91/23

(84) Benannte Vertragsstaaten:
AT DE FR GB IT

(56) Entgegenhaltungen:
CH-A- 560 042

(73) Patentinhaber: GEBRÜDER SULZER
AKTIENGESELLSCHAFT
Zürcherstrasse 9
CH-8401 Winterthur (CH)

(72) Erfinder: Frey, Otto
Wallrütistrasse 56
CH-8400 Winterthür (CH)
Erfinder: Bider, Kurt
Brunngasse 30
CH-8400 Winterthur (CH)

EP 0 263 292 B1

Jouve, 18, rue Saint-Denis, 75001 PARIS

**Beschreibung**

Die Erfindung betrifft ein Instrument zum Fräsen einer Krümmung in einen Femurknochen im Bereich des Calcar-Bogens, welches Instrument eine in eine in Längsrichtung des Markkanals verlaufende Bohrung einsetzbare Lehre aufweist, an der der Fräser unter einem Winkel angesetzt und entlang der er geführt ist.

Ein Fräsinstrument mit einer Lehre, die in eine in Längsrichtung des Markkanals verlaufende Bohrung eingesetzt wird, ist bekannt aus der CH-A-560042 oder der DE-OS 23 56 464 ; diese Lehre dient als Führung für einen Fräskopf, der unter einem unveränderlichen Winkel an die Lehre angesetzt ist. Mit diesem Fräser ist es möglich, in einer lateral-medialen Ebene einen geradlinig begrenzten dreieckigen Hohlraum aus dem Femurknochen im Bereich des Schenkelhalses auszufräsen. Mit diesem bekannten Instrument besteht jedoch nicht die Möglichkeit, eine bogenförmige Ausfräsung herzustellen, die beispielsweise der "Grenzlinie" zwischen spongiosem und kortikalem Gewebe im Bereich des Calcar-Bogens entspricht.

Aufgabe der Erfindung ist es, ein Instrument zu schaffen, mit dem ein derartiger Bogen möglichst genau und exakt gefräst werden kann. Diese Aufgabe wird mit der vorliegenden Erfindung dadurch gelöst, dass zum Fräsen einer in einer lateral-medialen Ebene verlaufenden Krümmung der Fräser von einer Spindel getragen ist, die mittels einer an der Lehre und an der Spindel verschwenkbar angeordneten Stange gelenkig an der Lehre befestigt ist, und dass die Spindel in einem Gleitschuh endet, der in einer in Längsrichtung der Lehre verlaufenden und einen Teilbereich der Lehre bildenden Gleitschuh geführt verschiebbar ist.

Mit der geführten Gleitbewegung, die im wesentlichen in Richtung der Längsachse des Instrumentes verläuft, lässt sich während des Fräsens eine bogenförmige Begrenzung des gefrästen Hohlraumes erzeugen. Die Form des Bogens, d.h. vor allem sein Radius, lassen sich variieren, wenn die Angriffspunkte der Anlenkung an der fräserspindel und an der Lehre höhenverstellbar sind, und/oder wenn die die Angriffspunkte der Anlenkung verbindende Stange austauschbar ist.

Um das "Fixieren" der Lehre in der Markkanalbohrung des Knochens zu erleichtern, ist es vorteilhaft, wenn die Gleitbahn gegen die Längsachse der Lehre geneigt ist. Durch diese Neigung der Gleitbahn wird die Achse der Lehre unterhalb der Gleitbahn gegenüber derjenigen oberhalb der Gleitbahn seitlich versetzt ; dadurch wird das Anliegen der Lehre am Knochen im Bereich des grossen Trochanters erleichtert.

Im folgenden wird die Erfindung anhand eines Ausführungsbeispiels im Zusammenhang mit der Zeichnung näher erläutert.

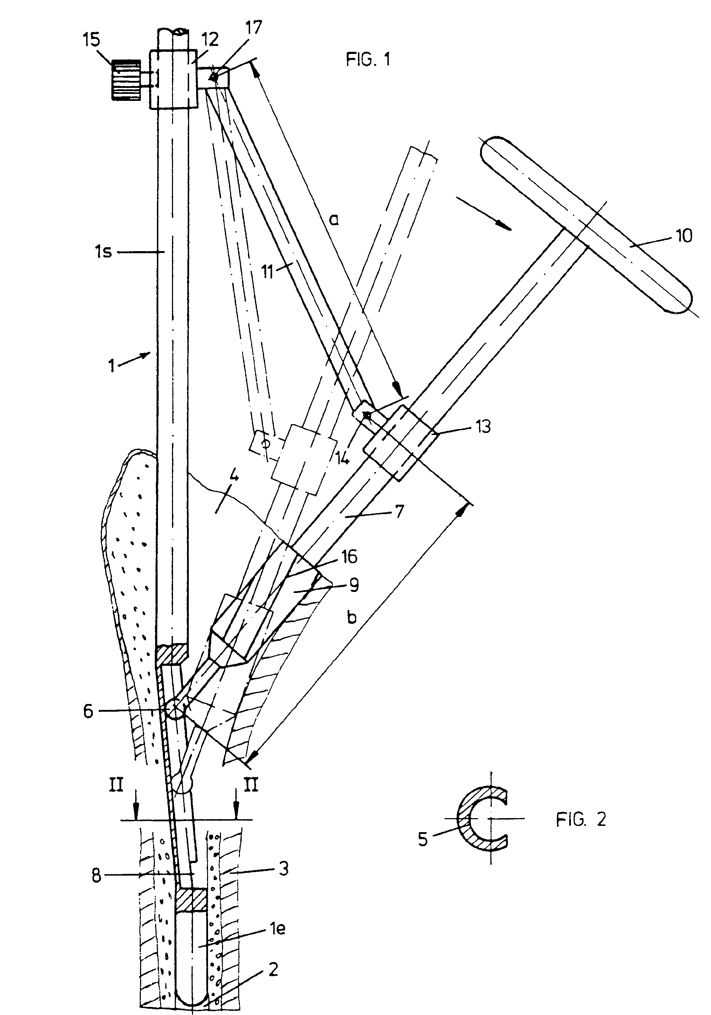

Fig. 1 zeigt das neue Instrument, eingesetzt in einen im Längsschnitt dargestellten Femurknochen ;

Fig. 2 ist der Schnitt II-II von Fig. 1.

Die Lehre 1 des neuen Instrumentes ist mit seinem freien Ende 1e, dessen Längsachse gegenüber der Längsachse des in einem nicht gezeigten Handgriff endenden, nach aussen führenden Lehrenstammes 1s seitlich versetzt ist, in eine künstlich erzeugte Bohrung 2 des Knochens 3 eingesetzt. Durch die Versetzung wird ein Anliegen des Stammes 1s der Lehre am Knochengewebe im Bereich des grossen Trochanters ermöglicht, obwohl die in der Spongiosa geschaffene Operationsöffnung 4 gegenüber der im Knochen 3 zentralen Bohrung 2 seitlich erweitert ist.

Das Ende 1e und der Stamm 1s sind miteinander durch ein hohles Zwischenstück von C-förmigem Querschnitt (Fig. 2) verbunden, das eine zu den Längsachsen geneigte Gleitbahn 5 bildet. In dieser Gleitbahn 5 ist ein kugelförmiger Gleitschuh 6 einer Spindel 7 gelagert, der in eine Erweiterung 8 der C-förmigen Oeffnung am unteren Ende der Gleitbahn 5 "eingefädelt" werden kann. Die Spindel 7, auf die der Fräser 9 von unten aufgeschoben und z.B. mittels zweier nicht gezeigter, seinen Mantel radial durchsetzender Schrauben im Klemmsitz gehalten oder an seiner oberen Stirnfläche mit der Spindel 7 verschweisst ist, endet in einem Handgriff 10.

Erfindungsgemäss sind die Spindel 7 und der Stamm 1s der Lehre gelenkig miteinander verbunden. Die Verbindung besteht aus einer Stange 11, die an ihrem Ende an je einer Schiebebüchse 12 und 13 lösbar befestigt ist, so dass sie gegebenenfalls gegen eine andere mit einer unterschiedlichen Länge a ausgetauscht werden kann. Die Büchsen 12 und 13 sind auf dem Stamm 1s der Lehre bzw. der Spindel 7 in Richtung der Längsachsen verschiebbar und werden durch Rändelschrauben 15 im Klemmsitz gehalten. Eine Verschiebung der Büchse 13 verändert dabei die Länge b zwischen dem Gleitschuh 6 und dem Anlenkpunkt 14 für die Stange 11 auf der Spindel 7. Aenderungen der Längen a und b sowie Verschiebungen der Büchse 12 auf dem Stamm 1s der Lehre verändern die Form und Lage des gefrästen Bogens, d.h. vor allem seinen Radius und seine Höhe relativ zu einem Bezugspunkt, beispielsweise der "Spitze" des grossen Trochanters.

Bei einem Einsatz des neuen Instrumentes empfiehlt sich beispielsweise folgendes Vorgehen :

Nach der Resektion des Gelenkkopfes werden zunächst auf konventionelle Weise die Bohrung 2 und anschliessend ebenfalls auf die übliche Art mit Hilfe einer Raspel in dem spongiosen Gewebe der Hohlraum 4 geschaffen. Mit Hilfe von Röntgen-Bildern ist vor dem Einsetzen des neuen Instrumentes in etwa der bogenförmige Verlauf der "Grenzlinie" zwischen Spongiosa und Kortikalis im Bereich des Calcar-Bogens festgestellt worden. Aufgrund der ermittelten Bogenform sind die Länge der Stange 11 und die Lage des Anlenkpunktes 14 auf der Spindel 7, also die grössen a und b, deren Verhältnis während der Operation möglichst konstant gehalten wird, festgelegt worden.

Nach dem Einsetzen der Lehre des Instrumentes mit ihrem Ende 1e in die Bohrung 2 wird der Stamm 1s an der lateralen, d.h. zum grossen Trochanter weisenden Begrezung des Hohlraumes 4, abgestützt und die Spindel 7 mit dem Fräser 9 in die tiefste Position gebracht, bei der Fräser 9 beginnen soll, den "innersten" Teil des Bogens zu schneiden. In dieser Stellung befindet sich der Gleitschuh 6 in seiner untersten Arbeitsposition.

Durch Drehen des Handgriffes 10 wird das spongiose Gewebe von den auf dem Mantel des Fräsers 9 angeordneten Fräskanten 16 herausgeschält, wobei zuerst die tiefsten Bereiche der Kanten 16 zum Einsatz kommen.

Durch eine Bewegung der Spindel 7 aus der strichpunktierten Stellung in die ausgezogen dargestellte Lage, wandert der Gleitschuh 6 auf der Gleitbahn 5 geführt nach oben, wobei die Bogenform der Ausfräsung dadurch entsteht, dass mit der Aufwärtsbewegung des Fräsers 9 der "schneidende Bereich" auf seinen Mantel ebenfalls nach oben wandert, wie dies in der Zeichnung durch das Anliegen unterschiedlicher Mantel-Höhen des Fräsers 9 am Knochen in der strichpunktierten und in der ausgezogen dargestellten Stellung verdeutlicht ist.

## Ansprüche

1. Instrument zum Fräsen einer Krümmung in einen Femurknochen im Bereich des Calcar-Bogens, welches Instrument eine in eine in Längsrichtung des Markkanals verlaufende Bohrung (2) einsetzbare Lehre (1) aufweist, an der der Fräser (9) unter einem Winkel angesetzt und entlang der er geführt ist, dadurch gekennzeichnet, dass zum Fräsen einer in einer lateral-medialen Ebene verlaufenden Krümmung der Fräser (9) von einer Spindel (7) getragen ist, die mittels einer an der Lehre (1) und an der Spindel (7) verschwenkbar angeordneten Stange (11) gelenkig an der Lehre (1) befestigt ist, und dass die Spindel (7) in einem Gleitschuh (6) endet, der in einer in Längsrichtung der Lehre (1) verlaufenden und einen Teilbereich der Lehre (1) bildenden Gleitbahn (5) geführt verschiebbar ist.

2. Instrument nach Anspruch 1, dadurch gekennzeichnet, dass die Gleitbahn (5) gegen die Längsachse der Lehre (1) geneigt ist.

3. Instrument nach Anspruch 1, dadurch gekennzeichnet, dass die Angriffspunkte (14, 17) der Anlenkung an der Fräserspindel (7) und an der Lehre (1) höhenverstellbar sind.

4. Instrument nach Anspruch 1, dadurch gekennzeichnet, dass die die Angriffspunkte (14, 17) der Anlenkung verbindende Stange (11) austauschbar ist.

## Claims

1. An instrument for cutting a curve in a thighbone near the calcar femorale, the instrument having a gauge (1) which is introducible into a bore (2) extending lengthwise of the marrow duct and to which the burr (9) is attached at an angle for guidance along it, characterised in that to cut a curve extending in a lateral-to-medial plane the burr (9) is carried by a spindle (7) articulated to the gauge (1) by way of a rod (11) disposed pivotally on the gauge (1) and spindle (7) ; and the spindle (7) terminates in a slide block (8) displaceably guided in a slideway (5) which extends lengthwise of the gauge (1) and is a partial zone thereof.

2. An instrument according to claim 1, characterised in that the slideway (5) is inclined relatively to the longitudinal axis of the gauge (1).

3. An instrument according to claim 1, characterised in that the places (14, 17) of articulation to the burr spindle (7) and gauge (1) are vertically adjustable.

4. An instrument according to claim 1, characterised in that the rod connecting the places (14, 17) of articulation is exchangeable.

## Revendications

1. Instrument pour fraiser une courbure à l'intérieur du fémur, dans la région de l'arc calcariforme, comportant un gabarit (1) à placer dans un perçage (2) s'étendant dans la direction longitudinale du canal médullaire, contre lequel la fraise (9) est placée sous un certain angle et le long duquel elle est guidée, caractérisé en ce que pour fraiser une courbure s'étendant dans un plan latéro-médial, la fraise (9) est portée par une broche (7) qui est fixée de manière articulée sur le gabarit (1), au moyen d'une tige (11) montée pivotante sur le gabarit (1) et sur la broche (7), et en ce que la broche (7) se termine par un patin (6) qui est guidé coulissant dans une voie de glissement (5) s'étendant dans la direction longitudinale du gabarit (1) et formant une région partielle du gabarit (1).

2. Instrument selon la revendication 1, caractérisé en ce que la voie de glissement (5) est inclinée vers l'axe longitudinal du gabarit (1).

3. Instrument selon la revendication 1, caractérisé en ce que les points d'attaque (14, 17) de l'articulation sur la broche de fraise (7) et sur le gabarit (1) sont réglables en hauteur.

4. Instrument selon la revendication 1, caractérisé en ce que la tige (11) reliant les points d'attaque (14, 17) est interchangeable.

FIG. 1

FIG. 2